# EUROPEAN PATENT APPLICATION

(11) **EP 2 957 610 A1**
(43) Date of publication of application: **23.12.2015**
(21) Application number: 15172879.7
(22) Date of filing: 19.06.2015
(51) Int. Cl.: C09J 7/02, C09J 11/00

(54) **DEBONDABLE ADHESIVE COMPOSITION**

(30) Priority: 20.06.2014 EP 14173246; 06.03.2015 EP 15157958
(71) Applicant: NITTO DENKO CORPORATION, Ibaraki-shi, Osaka 567-8680 (JP)
(72) Inventor: Schüwer, Nicolas, 1007 Lausanne (CH); De Visscher, Geofrey, 1071 Chexbres (CH)
(74) Representative: Hoffmann Eitle

(57) **Abstract**

The present invention provides an approach for making adhesive compositions and in particular pressure sensitive adhesive compositions that possess adjustable adhesion level allowing debonding on-demand in an easy, rapid and cost-efficient manner, wherein said approach can be easily applied to a wide variety of different adhesive systems without need for complex adaptation of the adhesive systems. The adhesive compositions of the present invention are characterized by an adhesive system and a debonding component, wherein the debonding component is an organic compound having a molecular weight of from 200 g/mol to 10000 g/mol, which debonding component contains two or more photodimerizable groups.

## Description

### 1. Technical Field of the Invention

The present invention is directed to adhesive compositions wherein the adhesive strength can be modulated during use. It pertains in particular to adhesive compositions that can be debonded during use.

The invention is also directed to a method for preparing and/or manufacturing the adhesive composition of the invention and to the use of the adhesive composition of the invention for laminating a sheet or a film, tapes and/or patches.

### 2. State of the art

US 2010/0316845 A1 relates to adhesive article which is debondable from substrates with the application of heat. This invention is based on a combination of a shape memory polymer backing having a temporary, deformed shape and pattern of latent projections coated with an amorphous pressure sensitive adhesive layer. The preparation of the adhesive sheets in accordance with this patent document is disadvantageous in that it requires complex protocol. Typical condition disclosed for triggering the debonding call for exposure at high temperature (≥100°C) for up to 15 min.

Takahara and coworkers reported reversible adhesion surface using polyelectrolyte brushes (Takahara, Atsushi, and Motoyasu Kobayashi. "*Precise Design of Antifouling, Intelligent Adhesion Surfaces through Polyelectrolyte Brush Immobilization*"). The adhesion strength in this study can be adjusted using ion exchange within the polyelectrolyte layer. The technology is however disadvantageous since it requires that the surfaces to be bonded are coated with polymer brushes.

WO 03/061720 A1 relates to adhesive systems and methods for activating or deactivating said adhesives. The patent document describes a skin adhesive system based on hydrocolloids in which adhesiveness can be activated or deactivated. The method for activating or deactivating the adhesiveness of an adhesive composition is based on the usage of an ionic solution which acts on the hydrocolloids in such a way that the physicochemical state thereof is modified.

WO 2001/005584 A1 relates to electrochemically debondable adhesives and coating compositions composed of a matrix moiety and an electrolyte moiety. The compositions undergo phase-separation upon the application of electric current leading to a decrease in the adhesion force. The invention is disadvantageous because it can only be used with conductive substrates.

WO 00/61692 relates to UV light switchable adhesive polymer capable of switching form tacky to less tacky state. The adhesive formulation is composed of a mixture functional polymer, using for instance norborn-2-ene methacrylate monomer, and a photoinitiator.

US 4,286,047 relates to pressure-sensitive adhesive susceptible to ultraviolet light-induced detackification. The adhesive system is based on a blend of oxirane ring-bearing component including an effective amount of onium salts ionic photoinitiator capable of promoting the polymerization of oxirane rings, whereby said adhesive is readily detackified by exposure to actinic radiation.

US 2011/0224593 A1 relates to switchable adhesives composition comprising a mixture of a polyacrylate adhesive; an unsaturated curable molecule (*i.e.* acrylate or methacrylate derivative), and a photoinitiator. This formulation is claimed to find use as adhesive medical dressing.

Boyne and coworkers (International Journal of Adhesion & Adhesives 21 (2001) 49) disclosed a pressure-sensitive adhesive capable of reducing its level of adhesion when exposed to visible light. The adhesive system reported is based on a mixture of a polyacrylate copolymer and a photoinitiator. The reduction in adhesion level was shown to be dependant on the intensity of light source used its wavelength spectra and the irradiation time. Similar formulation can find usage as medical grade adhesives (International Journal of Adhesion & Adhesives 19 (1999) 29).

Trenor et al. reported (The Journal of Adhesion 81 (2005) 213) the development of light-deactivable pressure sensitive adhesive compositions, wherein light-deactivation is accomplished *via* photodimerization of coumarin-functionalized repeating units of adhesive acrylate resins.

### 3. Problem solved by the invention

Having the state of the art summarized above, there remains a need for adhesive compositions and patches containing the same, which provide high adhesion level that can be lowered upon demand in order to facilitate the removal, and wherein the underlying approach is simple and flexible.

In particular, there is a need for an approach to provide debonding characteristics to a great variety of different adhesive systems, which does not require any significant modifications of the components constituting the various adhesive systems.

### 4. Summary of the Invention

The present invention meets this demand as it provides an approach for making adhesive compositions and in particular pressure sensitive adhesive compositions, that possess adjustable adhesion level allowing debonding on-demand in an easy, rapid and cost-efficient manner, wherein said approach can be easily applied to a wide variety of different adhesive systems without need for complex adaptation of the adhesive systems.

In particular, this problem is solved by the adhesive composition specified in appended claim 1. Preferred embodiments of the adhesive of the present invention are characterized in subsequent claims 2 to 10. The present invention further provides a layered product as specified in appended claim 11, which contains a layer made from the adhesive composition of the present invention. The methods of the present invention concern the manufacture of the adhesive composition of the present invention, the manufacture of the layered product of the present invention and the debonding of the products of the present invention. These methods are characterized in subsequent claims 12, 13 and 15.

### 5. Description of Figures

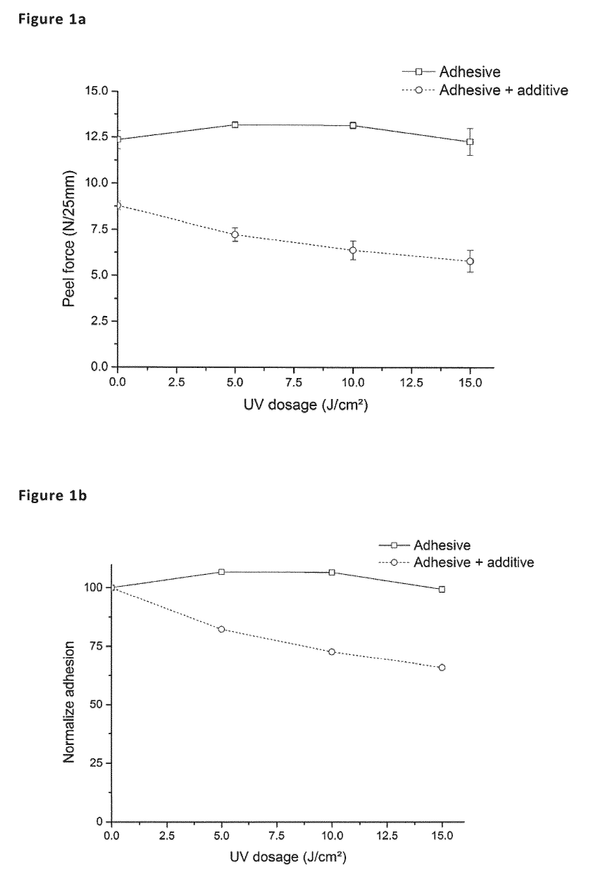

**Figure 1a****:** Evolution of peel forces values as function of UV dosage as determined in Example 1
**Figure 1b****:** Normalized of peel forces values as function of UV dosage as determined in Example 1

### 6. Detailed Description of the Invention

### 6.1 Definitions

The term **"switchable adhesive formulation"** is used in the context of the present invention to refer to an adhesive formulation, wherein the peel force can be decreased by the influence of electromagnetic irradiation. The term **"debondable adhesive formulation**" is used as an alternative expression to have the same meaning.

The term **"photodimerizable"** is used in the context of the present invention to refer to chemical groups or compounds, which can undergo a 2π+2π photodimerization reaction when being subjected to irradiation with light in the wavelength range of from 200 to 500nm.

The term ***"adhesive gel content value"*** refers to the value determined by the known method reported earlier (Vendamme R. and Eevers W. Macromolecules 46 (2013) 3395-3405).

The term ***"α transition"*** refers to the value determined by rheology. Rheological measurements were performed on a Discovery HR-2 hybrid rheometer (TA Instruments) in oscillation mode using parallel plate geometry. The α transition was measured by monitoring tan δ by scanning the temperature from -100°C to 150°C (ramp of 5 K/min) while imposing a constant strain of 0.4% and at a frequency of 1 Hz (linear regime conditions).

The term ***"peel force"*** or ***"adhesion level"*** refers to the force required to remove the tape once it is applied to a substrate. It can be measured using a Shimadzu Ag-X universal tensile testing machine equipped with 1000N force cell.

Unless specified otherwise, references to the ***molecular weight*** of a polymeric substance are to be understood as references to the weight average molecular weight, which is determined by Gel Permeation Chromatography (GPC) using Shimadzu GPC system equipped with a differential refractive index detector and calibrated with polystyrene standards.

Unless specified otherwise, amount ***indications in*** % are to be understood as indications in weight %.

The ***"acid value"*** is defined as the mass of potassium hydroxide (in mg) required to neutralized 1g of polycondensate, and is measured by direct titration using a standard ethanolic potassium hydroxide solution. Preferably, this measurement is carried out by dissolving a known amount of polycondensate in THF and titrating the obtained solution with 0.1N KOH ethanolic solution using an automatic titration device (Metrohm, Switzerland). This measurement is performed at 25°C.

The "***hydroxyl value"*** is defined as the mass of potassium hydroxide (in mg) equivalent to the hydroxyl content of 1g of polycondensate. It is measured by acetylation of the polycondensate followed by hydrolysation of the excess of acetic anhydride. The acetic acid formed is subsequently titrated with an ethanolic potassium hydroxide solution. Preferably the measurement is carried out by dissolving a known amount of polycondensate in THF, acetylating the mixture using a solution of acetic anhydride and 4-(dimethylaminopyridine) in THF (reaction time 15 min), quenching the excess of acetic anhydride (reaction time 30 min) with 4/1 mixture of THF/water (volume ratio); and running a back titration of the acetic acid formed during the quenching step with 0.5N KOH ethanolic solution using an automatic titration device (Metrohm, Switzerland). This measurement is performed at 25°C.

The term "***fatty acid***" is meant to refer to carboxylic acids having a long aliphatic chain, which may be saturated or may contain one or more unsaturations. The aliphatic chain may range from 4 to 30 carbon atoms, preferably from 6 to 28 carbon atoms, more preferably from 8 to 25 carbon atoms and even more preferably from 10 to 22 carbon atoms.

The term "***fatty alcohol***" is meant to refer to an aliphatic alcohol having a long aliphatic chain, which may be saturated or may contain one or more unsaturations. The aliphatic chain may range from 4 to 30 carbon atoms, preferably from 6 to 28 carbon atoms, more preferably from 8 to 25 carbon atoms and even more preferably from 10 to 22 carbon atoms.

The term "***bio***-***based***" is meant to refer to substances derived from renewable starting materials (as opposed to mineral oil-derived starting materials) by isolation and optionally one or more chemical transformations.

"***Suitability for topical administration to the human skin***" means in the context of the present application that the substance of interest must be authorized by European regulatory bodies for incorporation into medical preparations that are to be administered to the human skin. The final adhesive composition must be suitable for topical administration to the human skin. This means that the cross-linked polycondensate, the oil additive and any possibly present additional components must be suitable for administration to human skin. However, it does not mean that individual raw materials must be suitable for administration to human skin if these are consumed completely during the manufacturing process, or if residual raw material remains only at acceptably low levels or the product can be purified such that the purified product contains no or only acceptably low levels of potentially harmful raw materials.

Unless specified otherwise, references to *"**substituents**"* are meant to be references to an atomic group selected from fluorine, chlorine, bromine, iodine, hydroxy, -O-C(=O)-R¹, -C(=O)-R¹, -O-R¹, -R¹, -C(=O)-O-R¹, -N(R²)-C(=O)-R¹, -N(R²)-R1, -C(=O)-N(R²)-R¹, -O-C(=O)-O-R¹, -O-C(=O)-N(R²)-R¹, -N(R²)-C(=O)-O-R¹, -O-C(=N(R²))-R¹, -C(=N(R²))-O-R¹, -O-C(=N(R²))-N(R²)-R¹, - N(R²)-C(=N(R²))-O-R¹, -N(R²)-C(=N(R²))-N(R²)-R¹, wherein each of the above groups R¹ and R² may be independently selected from hydrogen, C₁₋₆ alkyl, C₂₋₆ alkenyl, C₂₋₆ alkynyl, C₆₋₁₀ aryl, 5 to 10 membered saturated, unsaturated or aromatic heterocycle containing 1 to 4 heteroatoms selected from O, N and S, wherein each of these groups R¹ and R² may itself optionally carry one to three substituents independently selected from halogen, C₁₋₆ alkyl, Q. ₆ alkoxy, -NH₂ and -NO₂.

Unless specified otherwise, references to ***"alkyl"*** groups are meant to be references to linear, branched or cyclic alkyl groups, provided the number of carbon atoms permits the formation of unsaturated bonds or cyclic systems.

In the context of the present application, the term ***"comprising"*** is meant to introduce an open list, such that further unmentioned members are not excluded. However, the use of "comprising" is intended to specifically characterize also the scenario, wherein no further unmentioned members are present. In other words, a disclosure of "comprising" is to be understood as a disclosure of "consisting of" as one distinct option.

In the context of the present application, the reference to a feature being a ***"preferred"*** feature is meant to indicate that it is preferred to combine this feature with other preferred features of the present application. All such combinations of preferred features with other preferred features are meant to be directly and unambiguously disclosed.

In the context of the present application, multiple limitations are to be considered in a cumulative manner.

### 6.2 Overview

The present invention solves the above problem by incorporating a debonding component into any kind of adhesive systems to thereby yield an adhesive composition that exhibits debonding characteristics.

The technical concept underlying the present invention is widely applicable. In particular, there are no restrictions concerning the adhesive system, to which the debonding component may be added. Moreover, there is a wide variety of debonding components available for use in the present invention.

There is no need to modify or adapt the adhesive system to the present invention in any way other than addition of the debonding component. In some instances, it may however be advantageous to take additional measures to ensure that the adhesive composition retains the state of a homogeneous mixture for a sufficiently long period of time to allow application of the adhesive composition, for instance by selecting and using suitable solvents, solubilizers, dispersants and/or emulsifiers and/or to adapt the chemical structure of the debondable additive accordingly.

Debonding is accomplished by irradiating the adhesive composition (or a patch or tape containing the same). Said irradiation initiates 2π+2π photodimerization reactions between two or more molecules of the debonding component. These reactions lead to an increase in stiffness of the adhesive composition, which, in turn, reduces the adhesion level of the adhesive composition.

### 6.3 Debonding Component

The debonding component consists of small organic compound that contains two or more photodimerizable groups. The meaning of "small" differs for the different embodiments of the present invention described below in connection with the components of Formula (I) and the components of Formula (II). However, the debonding component employed in the present invention is in any case small compared to the polymeric components used for debonding in the cited prior art of Trenor *et al.* as cited above.

The debonding component does not need to exhibit any adhesive characteristics itself. According to one embodiment of the present invention, the debonding component does not exhibit any adhesive characteristics.

### 6.3.1 Component of Formula (I)

According to one embodiment of the present invention, the debonding component consists of a compound characterized by the following general formula (I):

COR(-UN-PDM)ₙ (I)

In general formula (I), COR represents a core moiety selected from hydrocarbon groups having from 2 to 20, preferably from 3 to 8, carbon atoms and n free valencies forming covalent bonds to LIN or PDM. The structure of the hydrocarbon moiety may be saturated, unsaturated or aromatic, or combinations thereof. It may be cyclic or non-cyclic, linear or branched or combinations thereof.

The COR moiety may carry one or more substituents. According to a preferred embodiment in connection with any of the above or below embodiments, LIN or PDM may be bonded to COR via free valencies of such substituents.

The COR moiety may carry one or more heteroatoms, which are preferably selected from O, N, S, Si, P, and B. According to a preferred embodiment in connection with any of the above or below embodiments, COR contains one or more heteroatoms and LIN or PDM are bonded to COR via said heteroatoms.

COR moieties suitable for use in the present invention may for example be derived by abstraction of hydrogen atoms from the following compounds:

Polyhydric alcohols and sugars such as ethylene glycol, 1,3-propanediol, cyclohexandiol, hydroquinone, catechol, resorcinol, phloroglucinol, pyrogallol, hydroxyhydroquinone, tris(hydroxymethyl)benzene, tris(hydroxymethyl)benzene with three methyl or ethyl substituents bonded to the remaining benzene carbon atoms, isosorbide, isomannide, isoidide, glycerol, cyclohexane-1,2,4-triol, 1,3,5-cyclohexanetriol, pentane-1,2,3-triol, hexane-1,3,5-triol, erythritol, 1,2,4,5-tetrahydroxybenzene, threitol, arabitol, xylitol, ribitol, mannitol, sorbitol, inositol, fructose, glucose, mannose, lactose, 1,1,1-Tris(hydroxymethyl)propane, 1,1,1-Tris(hydroxymethyl)ethane, di(trimethylolpropane), trimethylolpropane ethoxylate, 2-Hydroxymethyl-1,3-propanediol, Pentaerythritol allyl ether and pentaerythritol.

Polyamines such as ethylenediamine, cadaverine, diethylenetriamine, putrescine, spermidine, spermine, homospermidine, triethylenetetramine, propane-1,2,3-triamine, cyclohexane-1,3,5-triamine, benzene-1,3,5-triamine, benzene-1,2,4-triamine, tris(2-aminoethyl)amine, 1,3,5-triazine-2,4,6-triamine, 2-(aminomethyl)propane-1,2,3-triamine, ethane-1,1,2,2-tetraamine, butane-1,1,4,4-tetraamine, pyridine-2,3,5,6-tetraamine.

Compounds with mixed functional groups selected from hydroxyl, amino, carboxyl, and/or amido groups such as lysine, arginine, ornithine, glycine, homoglycine, serine, proline, threonine, tyrosine.

Each LIN group represents a linker moiety independently selected from -R³-, -O-C(=O)-, -C(=O)-, -O-, -C(=O)-O-, -N(R²)-C(=O)-, -N(R²)- , -C(=O)-N(R²)-, -O-C(=O)-O-, -O-C(=O)-N(R²)-, -N(R²)-C(=O)-O-, -O-C(=N(R²))-, -C(=N(R²))-O-, -O-C(=N(R²))-N(R²)-, - N(R²)-C(=N(R²))-O-, -N(R²)-C(=N(R²))-N(R²)-, and combinations of two or more of these groups, wherein R¹ and R² are as specified above and R³ represents a divalent group selected from C₁₋₆ alkylene, C₂₋₆ alkenylene, and C₂₋₆ alkynylene. Alternatively, LIN may be absent. The absence of LIN is an advantageous option especially if COR and PDM contain functional groups that can be coupled with each other, such as a carboxylic acid group and a hydroxyl or amino group and if the direct coupling of two or more PDM groups to COR is sterically possible.

The effects of substitution on the active photodimerizable moiety are known to the person skilled in the art; and the interested reader can refer to "Elements of organic photochemistry" by Dwaine O. Cowan and Ronald L. Drisko (Plenum Press, 1976); Kuzuya et al. The Bulletin of the Chemical Society of Japan 59 (1986, 1379; Hoyer et al. Chemical Physics Letters 443 (2007) 107; or Kaur et al. Polymer Chemitry 5 (2014) 2171; for instance.

In a preferred embodiment in connection with any of the above or below embodiments, the LIN and/or the COR group are selected such that they do not affect in a negative way the photodimarization reaction, *i.e.* that the conversion rate of the photodimerization of the debonding component of the present invention is comparable (in terms of molar amounts of photodimerizable groups) to the photodimerization of the single compounds from which the photodimerizable groups are derived when applying the same irradiation conditions; preferably, this means that the conversion rate is no less than 75% of the conversion rate of the underlying single compound.

In a preferred embodiment in connection with any of the above or below embodiments, the LIN and/or the COR group facilitate the photodimerization reaction by improving, for instance stacking of adjacent components (I) e.g. due to the presence of flat aromatic groups.

Each PDM group represents a photodimerizable group, which is typically an ethylenically unsaturated group, which is bonded to one or more delocalized π-electron systems such as an aromatic or heteroaromatic ring system or a moiety that allows conjugation due to the presence of further π-electron pairs of atomic groups or atoms such as further ethylenically unsaturated bonds, carbonyl groups, and/or heteroatoms with free electron pairs such as nitrogen, wherein said bonding permits delocalization of the π-electrons of the ethylenically unsaturated group via conjugation with the adjacent π-electron system. Of course, the π-conjugation may also take place between the ethylenically unsaturated bond of the photodimerizable group and a suitable atomic group of the directly adjacent COR moiety (if LIN is absent) or LIN moiety (if it is present).

The photodimerizable group PDM is preferably derived from a compound selected from coumarin, stilbene, styrylpyridine, diphenylhexatriene, naphthalene, anthracene, pyridoquinoline, cinnamic acid, thymine, maleimide, fumarate and substituted forms thereof, wherein one free valency is generated by abstracting one of the hydrogen atoms of these compounds to provide the required free valency for covalent bonding to the LIN or COR group.

In a preferred embodiment in connection with any of the above or below embodiments, PDM is derived from a compound selected from coumarin and cinnamic acid and most preferably cinnamic acid. Thus, it is also preferred to use cinnamic acid carrying one or more substituents, especially electron withdrawing substituents as described below.

*n* represents an integer of from 2 to 10 preferably from 3 to 6 .

The molecular weight of the debonding component of formula (I) is typically in the range of from 200 g/mol to 3000 g/mol, preferably in the range of from 300 to 1500. The parameter "n" as well as the identity of the COR, LIN and PDM moieties are to be selected accordingly.

In a particularly preferred embodiment in connection with any of the above or below embodiments, COR is derived from a tri-, tetra or hexafunctional alcohol or amine compound and PDM is derived from cinnamic acid, which is directly bonded to COR via ester or amide formation with the hydroxyl or amino groups of COR, respectively.

According to a preferred embodiment in connection with any of the above or below embodiments, PDM is a group, preferably a group derived from coumarin, stilbene, styrylpyridine, diphenylhexatriene, naphthalene, anthracene, pyridoquinoline, cinnamic acid, thymine, maleimide, fumarate, which further carries one or more electron-withdrawing substituents.

Electron withdrawing groups are understood in the context of the present invention to be groups that exhibit an inductive and/or mesomeric effect that reduces the electron density at the active center, typically the ethylenically unsaturated group. These are preferably substituents that have a positive Hammett substituent parameter σ(para) for the standard benzoic acid dissociation reaction employed by Hammett, such as -F (Hammett, Louis P. (1937). "The Effect of Structure upon the Reactions of Organic Compounds. Benzene Derivatives". J. Am. Chem. Soc. 59 (1): 96). For a compilation of Hammett substituent parameters σ(para), please see D. H. McDaniel and H. C. Brown, J. Org. Chem., 23, 420 (1958). More preferably, one or more substituent(s) is/are present, which exhibit a substituent parameter σ(para) greater than 0.2 such as -Cl, -Br and -I. Even more preferably, the substituent parameter σ(para) is greater than 0.4. Such particularly preferred substituents include-CN, -CF₃ and -NO₂.

The electron withdrawing substituents may be bonded to the delocalized π-electron system or to the active center, typically the ethylenically unsaturated group. Of course, the presence of substituents is acceptable in the context of the present invention only if the substituent does not prevent the photodimerization reaction for other (*e.g*. steric) reasons. 4-Chloro-α-cyanocinnamic acid, the structure of which is depicted below, may be regarded as an model compound for an PDM group that carries an electron withdrawing group, namely the cyano group, at the active center. The 4-Chloro substituent acts as another electron withdrawing group. Of course, to form a PDM group according to the present invention, this compound must be bonded to COR or LIN, *e.g.* via an ester linkage.

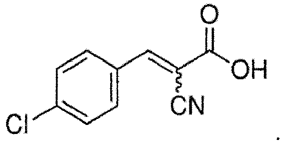

According to a preferred embodiment in connection with any of the above or below embodiments, the COR moiety may carry one, two or three, more preferably one, solubilizing groups SOL in addition and separate from the (-LIN-PDM) groups. Said SOL groups may be directly bonded to free valencies of the COR moiety or they can be bonded to free valencies of the COR moiety via LIN groups as defined above.

According to another embodiment of the present invention in connection with any of the above or below embodiments, the PDM group may carry one or more electron withdrawing groups and additionally one, two or three solubilizing groups SOL as defined in more detail below.

The SOL group may advantageously be selected from linear or branched alkyl groups with 1 to 24 carbon atoms or linear or branched alkenyl groups with 2 to 24 carbon atoms and poly(C₂₋₆ alkylene ether) groups with 1 to 6 repeating units. More preferably, the alkyl group is branched and has from 6 to 24 carbon atoms, as 2-ethylhexyl group for instance. Also more preferred is the use of a polyether solubilizing group selected from polyethylene glycol (PEG), poly(tetramethylene ether) glycol (PTMEG) and polypropylene glycol (PPG).

It is preferred that all PDM groups (including substituents) of a debonding component of formula (I) are the same.

Particularly preferred components of Formula (I) are shown below: 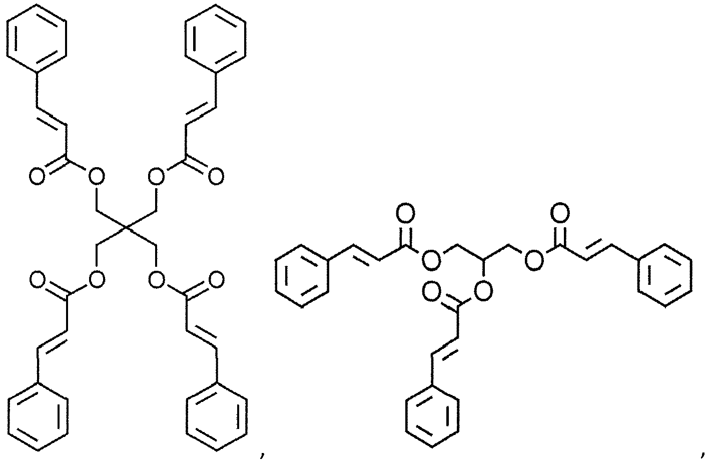 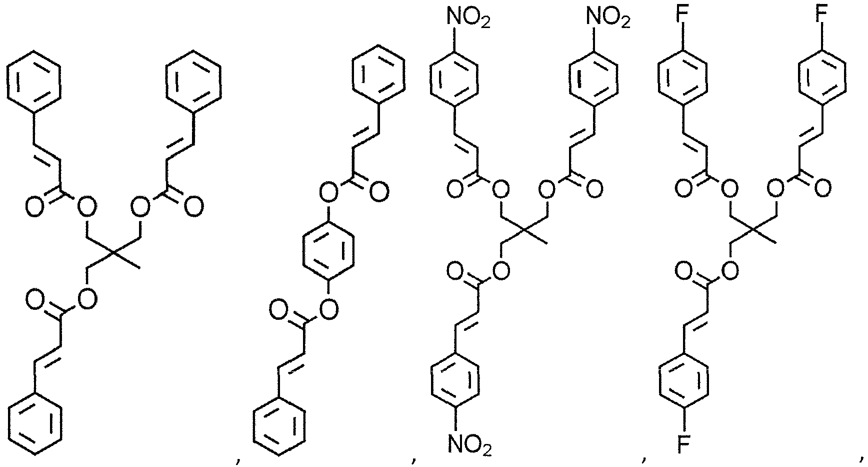 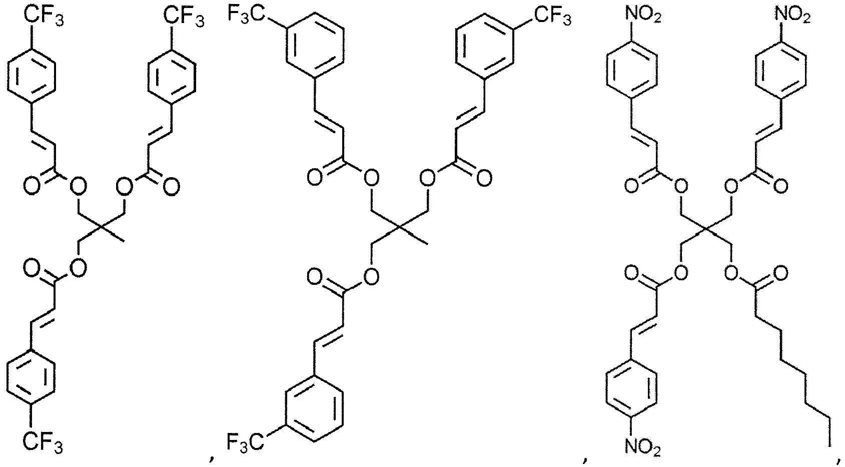 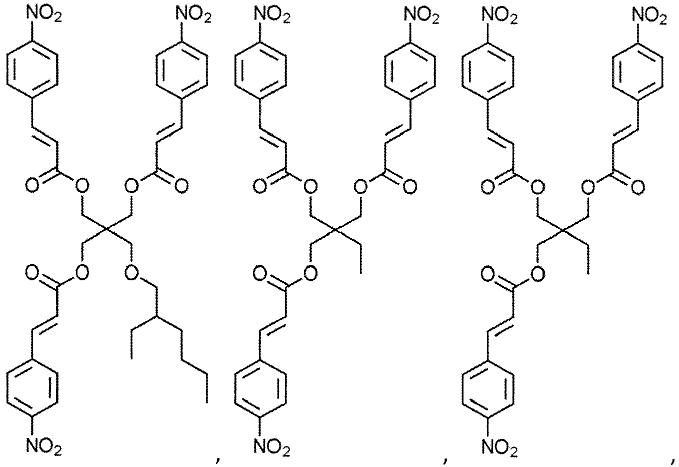 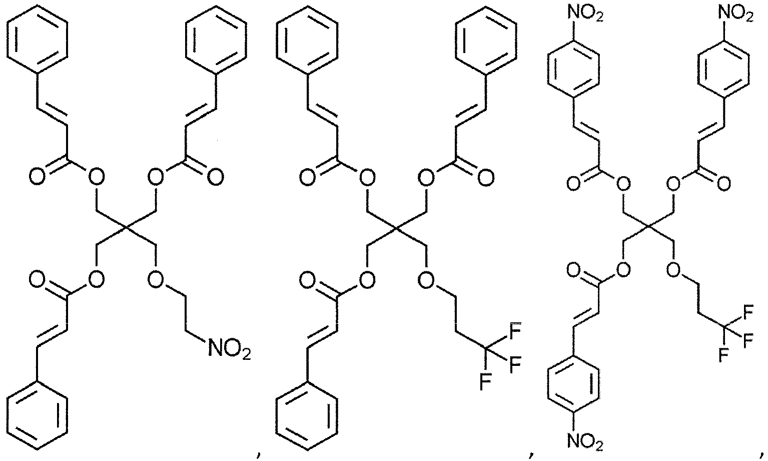 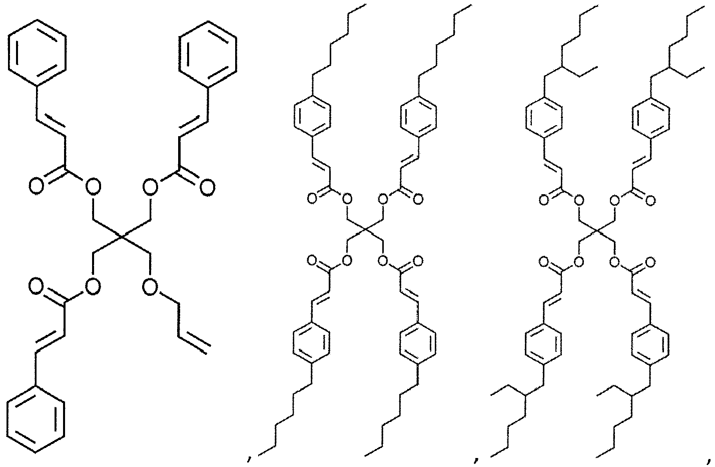 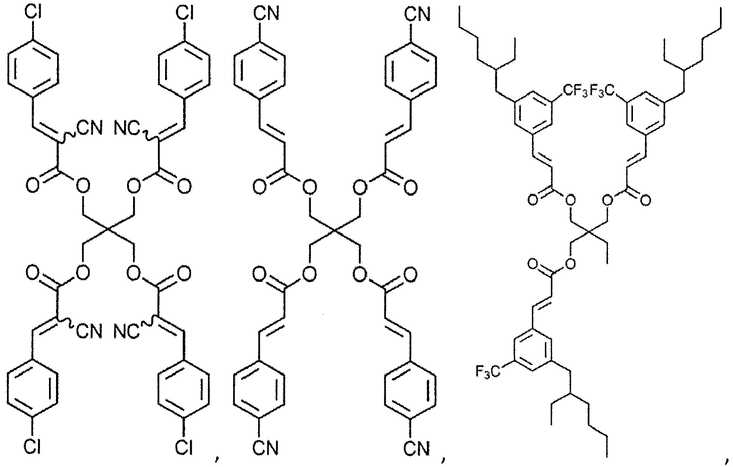 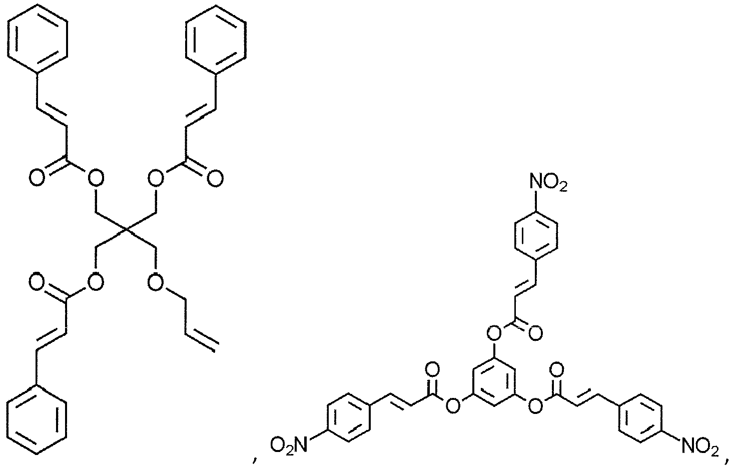 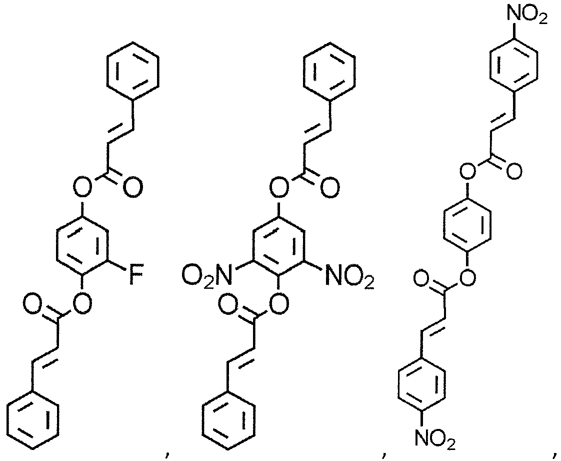 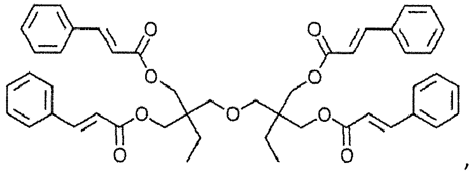 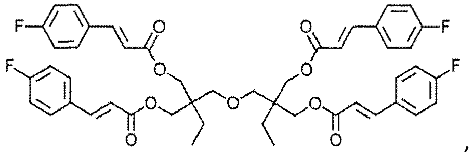 and

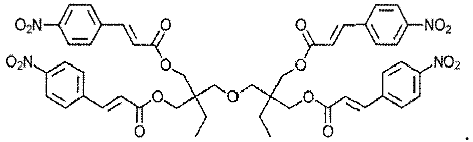

Further particularly preferred compounds of Formula (I) are the polyethylene glycol-based compounds shown below: 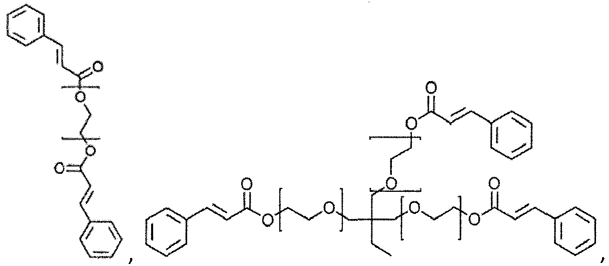 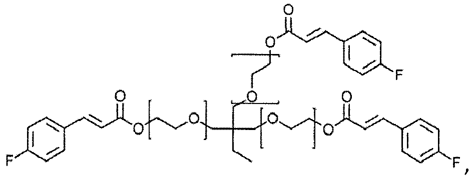 and

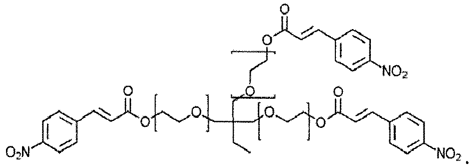

For the above polyethylene glycol-based compounds, there is no particular limitation to the number of oxyethylene moieties within the COR moiety other than the above limitations to the number of carbon atoms in the COR moiety and to the total molecular weight of the component.

As a consequence of the above, it is less preferred to use compounds of formula (I) that carry a substituent or functional group increasing the electron density at the active center. This may be a substituent of the PDM group having a negative Hammett substituent parameter σ(para) or it may be a functional group linking COR or LIN to PDM, which increases the electron density at the adjacent active center. Such functional linking groups are, for instance, amide bonds and imine bonds.

Specific compounds with increased electron density, which are less preferred, are listed below:

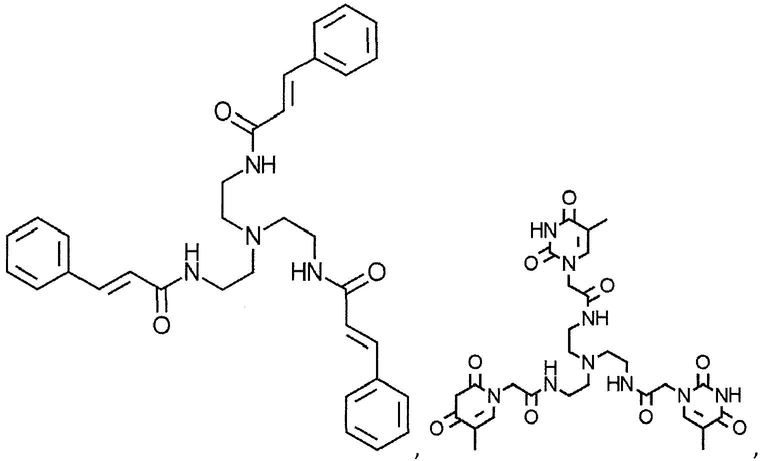

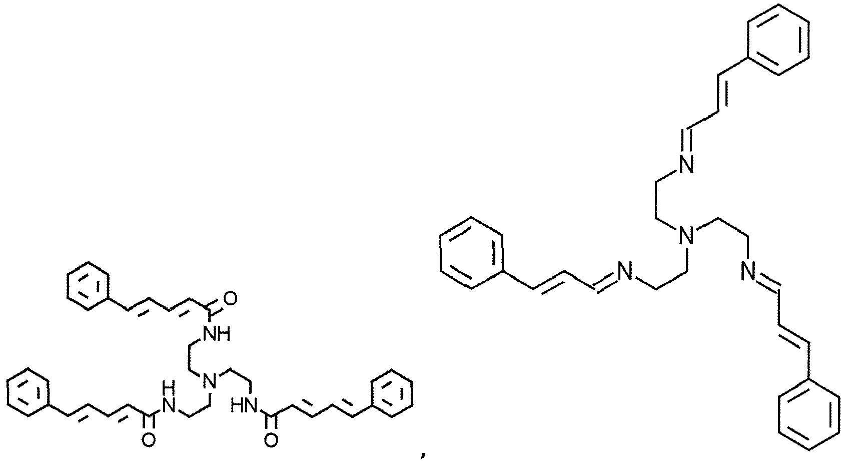

### 6.4 Component of Formula (II)

Further debonding components for use in the present invention may be selected from oligomeric compounds which contain two or more, preferably from 2 to 20 repeating units of general formula (II):

-(REP (-LlN-PDM))- (II)

wherein the molecular weight of the oligomeric compound of formula (II) is in the range of from 200 g/mol to 12000 g/mol, preferably from 500 g/mol to 10000 g/mol, more preferably in the range of from 550 g/mol to 6000 g/mol and most preferably 600 g/mol to 4000 g/mol.

The oligomeric compounds containing the repeating units of general formula (II) may be terminated by hydrogen atoms or any substituent group as specified above.

Moreover, the oligomeric compounds containing the repeating units of general formula (II) may contain divalent groups as additional repeating groups. The scope of such additional repeating units is not particularly restricted, provided the additional repeating units contain two atomic groups that can be covalently bonded to the repeating units of general formula (II).

REP represents a repeating unit core moiety derived from a monomeric compound selected from amino acids, saccharides, including cyclodextrin-forming saccharides, vinylacohol and preferably from lysine monomers.

In another embodiment, REP represents a linear or branched C₂₋₆ alkylene ether repeating unit, preferably a repeating unit selected from an ethylene ether repeating unit, a propylene ether repeating unit and a tetramethylene ether repeating unit. The resulting polyether preferably contains from 2 to 300 repeating units, more preferably from 10 to 250 repeating units and most preferably from 50 to 230 repeating units.

The -PDM moieties may be bonded to the polyether via the termini and/or be bonded to the polyether backbone. Bonding to the polyether backbone may for instance be accomplished via functionalized alkylene ether repeating units, which carry a side chain with a functional group, to which the -PDM moiety can be bonded. For instance, the side chain of the polyether repeating unit may carry a hydroxyl or carboxyl group, which can react with a carboxyl or hydroxyl group, respectively, to from an ester linkage as LIN moiety.

It is not necessary that every polyether repeating unit carries a PDM group. Instead, it is preferred to combine PDM group-carrying repeating units with other C₂₋₆ alkylene ether repeating units. In this embodiment, it is preferred that the repeating units carrying a PDM group are present in a relative amount of 0.05 to 60 mol%, preferably 1 to 35 mol% and most preferably 5 to 25 mol%, based on the total molar amount of all repeating units of the polyether.

LIN represents a linker which is as specified above with respect to general formula (I).

PDM is a photodimerizable group which is as specified above with respect to general formula (I).

According to one embodiment in combination with any of the above and below embodiments, the debonding component is an oligomer of lysine, to which cinnamic acid is linked via an amide bond. According to a preferred embodiment in combination with any of the above and below embodiments, the debonding component is an oligomer of serine and/or threonine and/or tyrosine and/or cysteine to which cinnamic acid is linked via an ester bond or thioester bond, respectively. According to a particularly preferred embodiment of any one of these embodiments, the debonding component is characterized by 2 to 30, and most preferably 4 to 20 repeating units consisting of the lysine-cinnamic acid amide, that are bonded to each other via peptidic linkages.

According to a preferred embodiment in combination with any of the above and below embodiments, the oligomer is terminated by two groups that are independently selected from hydrogen atoms, C₁₋₆ alkyl groups and C₁₋₆ alkanoyl groups.

As explained above with respect to the compounds of formula (I), it also applies to the compound of formula (II) that the effects of substitution on the active photodimerizable moiety are known to the person skilled in the art; reference is again made to "Elements of organic photochemistry" by Dwaine O. Cowan and Ronald L. Drisko (Plenum Press, 1976) ; Kuzuya et al. The Bulletin of the Chemical Society of Japan 59 (1986) 1379; Hoyer et al. Chemical Physics Letters 443 (2007) 107; or Kaur et al. Polymer Chemitry 5 (2014) 2171.In a preferred embodiment in connection with any of the above or below embodiments, the LIN and/or the COR group are selected such that they do not affect in a negative way the photodimerization reaction, *i.e.* that the conversion rate of the photodimerization of the debonding component of the present invention is comparable (in terms of molar amounts of photodimerizable groups) to the photodimerization of the single compounds from which the photodimerizable groups are derived when applying the same irradiation conditions; preferably, this means that the conversion rate is no less than 75% of the conversion rate of the underlying single compound.

In a preferred embodiment in connection with any of the above or below embodiments, the LIN and/or the COR group facilitate the photodimerization reaction by improving, for instance stacking of adjacent components (I) e.g. due to the presence of flat aromatic groups or affecting in a beneficial manner the electron density on the PDM groups.

### 6.5 Adhesive Systems

As noted above, there is no particular limitation regarding the adhesive systems that can be used for making the adhesive compositions of the present invention. Adhesive systems may in particular be pressure-sensitive adhesive systems. Typical representatives of such systems, which are all useful for the present invention, are acrylics, rubbers, silicones, polyurethanes, polyesters, polyethers and EVA systems. However, the present invention may also be applied to adhesive systems other than pressure-sensitive adhesive systems, such as sealants, hot-melts, glue and hydrocolloid systems.

Further preferred adhesive systems for use in the present invention are those described in the co-pending European Patent Applications Nos. 13 185 924.1, 13 198 932.9 and 13 198 936.0.

For instance, any of the pressure-sensitive adhesive systems described in "Adhesion Science and Engineering" edited by M. Chaudhury and A.V. Pocius, Elsevier B.V., 2002; "Pressure-sensitive Adhesives and Applications" by I. Benedek, 2nd Ed., Marcel Dekker Inc., 2004; "Technology of Pressure-Sensitive Adhesives and Products" edited by I. Benedek and M.M. Feldstein, CRC Press, 2009; as well as Zbigniew Czech and Agnieszka Kowalczyk (2011) "Pressure-Sensitive Adhesives for Medical Applications", Wide Spectra of Quality Control, Dr. Isin Akyar (Ed.), ISBN: 978-953-307-683-6, InTech, Available from: http://www.intechopen.com/books/wide-spectra-of-quality-control/pressure-sensitive-adhesives-for-medicalapplications.

Preferred adhesive systems are acrylics, silicones, polyurethanes, hydrocolloids and polyesters.

According to a preferred embodiment in connection with any of the above or below embodiments, the adhesive system is based on a polymer, which also contains repeating units carrying a PDM group as defined above. That is, the composition contains a debonding compound with PDM groups of formula (I) and/or (II) together with an adhesive polymer, which itself also carries PDM groups. Typically, said PDM group of the adhesive polymer may be present in a side-chain of a repeating unit of the polymer, such that a group -PDM or a group -LIN-PDM is bonded to the polymer backbone, wherein LIN and PDM have the meanings defined for component (I) above. Bonding of the side chain to the polymer backbone may for instance be accomplished via ester linkages. This can be easily accomplished in (meth)acrylate-based polymers. However, the introduction of PDM groups is contemplated also for the other systems listed above. A possible schematic structural representation of an adhesive polymer according to this embodiment is shown below relying, for instance, on a (meth)acrylate-based polymer: 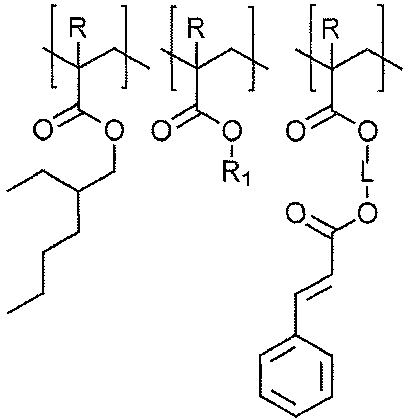 Wherein R is -H or -CH₃; R₁ is -H, -CH₃ or -ⁿButyl, for instance; and L can be -CH₂-CH₂-.

Another example of a preferred acrylate-based polymer according to this embodiment is shown below:

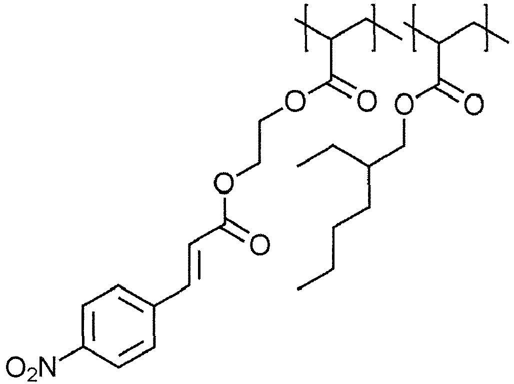

An example of an adhesive system other than an acrylate-based system is shown below:

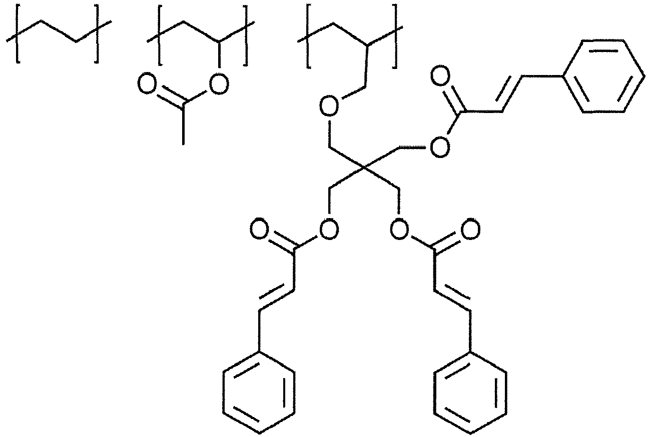

Such polymers can be made either via direct polymerization of the appropriate monomers (one of which carrying the PDM group) or via post polymerization reactions of a reactive polymer with a suitable starting material yielding the desired PDM group. A preferred PDM group of this embodiment of the invention is the cinnamic acid-derived group Phenyl-CH=CH-CO- which may optionally carry substituents (e.g., -NO₂ as described above for formula (I).

Introduction of PDM groups into the adhesive system increases the density of PDM groups in the system and therefore accelerates the overall light induced switch. In this embodiment with PDM groups in the polymer and in the debonding additive, three different types of photodimerization reaction are possible, namely polymer-polymer, additive-additive and polymer-additive.

In this embodiment, the PDM group of the adhesive polymer is preferably the same as the adhesive group of the debonding compound.

In this embodiment, it is preferred that the repeating units carrying a PDM group are present in a relative amount of 0.05 to 60 mol%, preferably 1 to 35 mol% and most preferably 5 to 25 mol%, based on the total molar amount of all repeating units of the polymer. The polymer is preferably a random copolymer although block-copolymers are not strictly excluded.

In this embodiment, it is preferred that the weight ratio of debonding additive to adhesive polymer containing PDM groups is from 0.5 to 50, preferably 1 to 25 and more preferably 5 to 15.

In this embodiment, it is preferred that the average molecular weight of the polymer containing PDM groups is from 20 000 to 5 000 000, preferably 50 000 to 1 000 000 and more preferably 80 000 to 350 000 g/mol.

It is also preferred to incorporate a light permeation enhancer to accelerate the photodimerization process. Such substances may for instance be selected from transparent microparticles, which may consist of glass, poly(meth)acrylates, etc.

### 6.6. Manufacture of Adhesive Composition

The adhesive composition of the present invention can be manufactured by admixing the debonding component to a pre-existing adhesive system, *e.g.* a commercially available adhesive system. Any type of admixing technique may be used, which is appropriate for the viscosity of the adhesive system. If the viscosity is too high, it may be considered to dilute the adhesive system in a suitable solvent or to carry out the admixing procedure at elevated temperature.

Alternatively, the adhesive composition of the present invention may be manufactured by mixing the debonding component with the remaining components of the adhesive system while manufacturing the adhesive system. In this case, there is no particular limitation to the relative order of mixing the components.

This alternative procedure may be advantageous for instance in those cases, wherein the final adhesive system is obtained by cross-linking an intermediate adhesive composition only after it is coated on a suitable support. In such instances, it is advantageous to incorporate the debonding component into the intermediate adhesive composition before the intermediate adhesive composition. This can be done either by mixing together at the same time the debonding component with some or all of the other components, or by admixing the debonding component to a pre-fabricated intermediate adhesive composition.

As noted above, it may be advantageous to take appropriate action to avoid demixing of adhesive system and debonding component. The adhesive composition of the present invention is advantageously at least kinetically stable such that demixing is avoided during manufacture, storage and application. Once the adhesive composition is applied to a substrate, it is usually immobilized by cross-linking and/or evaporation of solvent. Demixing will usually no longer occur after immobilization. Hence, stabilization of the adhesive composition is advantageously sufficient to prevent demixing during the time period between manufacture and immobilization.

Suitable means for preventing demixing are the use of suitable solvents such as toluene, xylene or ethylacetate, solubilizers and/or emulsifiers such as propylene glycol and polyethylene glycol esters.

### 6.7. Medical Tape and Patch

### 6.7.1 Backing

To prepare a medical tape or patch, the adhesive composition of the present invention is laminated on a backing. Suitable backing materials may, for instance, be selected from woven and non-woven materials, wherein preferred woven backing materials are selected from cotton, nylon, rayon or a mixture thereof; and wherein preferred non-woven materials are preferably selected from polyethylene terephthalate (PET), polyethylene (PE), polyurethane (PU), polyvinyl chloride (PVC), polypropylene (PP), wood pulp, cellulose, starch and/or polylactic acid (PLA). Suitable backing materials do not absorb electromagnetic radiation at wavelength needed for the photodimerization process.

In a preferred embodiment in combination with any of the above or below embodiments the adhesive composition of the invention is laminated onto a biobased non-woven having one or more of the following properties: weight: 15 - 100 g/m², thickness: 0.10 - 1 mm, maximum tensile strength: 30 - 150N/50mm and maximum elongation 15-70%. It is more preferred to use a backing material fulfilling two of these properties, even more preferably three of these properties and most preferably all four of these properties.

In a preferred embodiment the backing material used can withstand radiation induced curing of the adhesive formulation without visible and/or mechanical change.

In a preferred embodiment the backing material used can withstand sterilization process without visible changes (e.g. no yellowing).

### 6.7.2 Patch-related Features

The patch of the present invention has a construction and layer structure corresponding to that of a conventional patch. It is characterized by the presence of the adhesive composition of the present invention instead of the presence of a standard pressure-sensitive adhesive.

Hence, the patch of the present invention may comprise one or more of the following features: structure consisting of non-adhesive area and adhesive area; structure comprising non-adhesive fluid absorbent material and adhesive area; structure comprising moisture regulation material/compound and adhesive area; structure containing active agent reservoir material and adhesive area.

The present invention also encompasses patches, wherein adhesive thickness is not the same everywhere in the patch. This can be, for instance, patches, wherein the adhesive is thicker or thinned at the edge.

Further applications of the adhesive composition of the present invention are in the fields of ostomy care, skin traction adhesives, surgical operation films and electrode adhesives. The conventionally used materials may be adapted in accordance with the present invention by incorporating a debonding component as described above, without need for any further modifications.

### 6.7.3 Sterilization

The medical tape or patch of the present invention can also be sterilized if the final application requires it. Any known method of sterilization can be used provided it does not affect adhesive strength by initiating the debonding reaction.

### 6.8 Non-medical Uses

Non-medical uses of the adhesive composition of the present invention are not particularly limited. They include in particular uses of the adhesive composition of the present invention in masking tape, protective tape, and in semi-conductor immobilization.

For any of these uses, the adhesive composition of the present invention may be used as a replacement of any conventionally used adhesive. In a preferred embodiment in combination with any of the above or below embodiments the adhesive composition of the present invention is based on the same adhesive system, which is already used in the application of interest, so that the resulting masking tape, protective tape, *etc.* differs from the underlying conventional one only by the addition of the debonding component.

The manufacture of such non-medical application products is also not particularly restricted. Any conventionally employed technique may be used. According to a preferred embodiment in combination with any of the above or below embodiments, the product is a layered product, which is manufactured by coating the adhesive composition of the present invention onto a backing material. Any conventionally employed backing material may be used, including for instance the backing materials mentioned above and especially polyethylene terephthalate (PET) and polyethylene (PE)..

Alternatively, the adhesive composition of the present invention may be coated onto a release liner surface consisting *e.g*., of silicone-coated paper. The resulting layered structure may subsequently laminated with a backing material, wherein the backing material is preferably selected from the backing materials mentioned above.

Semi-conductor immobilization is meant to refer at least to the immobilization of silicon wafers in the clean room process.

### 6.9 Debonding

In use, the adhesive composition of the present invention and/or a layered product such as a medical tape or patch or a non-medical masking tape or protective tape, which comprises the adhesive composition of the present invention is applied to a substrate surface. For instance, the medical tape or medical patch of the present invention is typically applied to the human skin.

Debonding of the adhesive composition and/or layered product of the present invention can be accomplished by irradiating the adhesive composition and/or layered product of the present invention with light having a wavelength in the range of from 200 to 500 nm.

It is of course possible and preferred to optimize the debonding effect by adjusting the wavelength of the irradiated light to the absorption characteristics of the photodimerizable group contained in the debonding component used.

In a preferred embodiment in combination with any of the above or below embodiments, light irradiation is carried out with a light intensity and/or for a period of time, which is sufficient to reduce the peel force of the adhesive composition to the substrate surface by 40% or more, more preferably by 60% to 95%.

Upon irradiation, the adhesive composition is typically removed from the substrate surface by applying a sufficiently high peel force.

### 6.10 Examples

### 6.10.1 Example 1

An adhesive composition according to the present invention is prepared by mixing 90 wt.% of a commercial adhesive system based on DOW MD 7/4502 silicone adhesive with 10 wt.% of the debonding compound shown by the formula below:

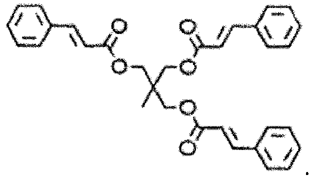

For comparison, an adhesive composition consisting of 100 wt.% of the same commercial adhesive system based on DOW MD 7/4502 silicone adhesive was considered.

Figure 1 displays the evolution of the peel force value, measured on a Bakelite substrate. From Figure 1 one can see that the formulation containing the additive undergoes a decrease in its peel force value of one third upon UV exposure, whereas the pure adhesive of the comparative sample does not show any significant adhesion variation when exposed to the same irradiation conditions.

## Claims

1. Adhesive composition comprising an adhesive system and a debonding component, wherein the debonding component is an organic compound having a molecular weight of from 200 g/mol to 12000 g/mol and preferably from 200 g/mol to 10000 g/mol, which debonding component contains two or more photodimerizable groups.

2. Adhesive composition according to claim 1, wherein the debonding component is a compound **characterized by** the following general formula (I):
COR(-LIN-PDM)ₙ (I)
wherein COR represents a core moiety selected from hydrocarbon groups having from 2 to 20 carbon atoms and n valencies;
each LIN group represents a linker moiety independently selected from a covalent bond,-R³-, -O-C(=O)-, -C(=O)-, -O-, -C(=O)-O-, -N(R²)-C(=O)-, -N(R²)- , -C(=O)-N(R²)-, -O-C(=O)-O-, -O-C(=O)-N(R²)-, -N(R²)-C(=O)-O-, -O-C(=N(R²))-, -C(=N(R²))-O-, -O-C(=N(R²))-N(R²)-, - N(R²)-C(=N(R²))-O-, -N(R²)-C(=N(R²)-N(R²)-, and combinations of two or more of these groups, wherein each of the above groups R¹ and R² may be independently selected from hydrogen, C₁₋₆ alkyl, C₂₋₆ alkenyl, C₂₋₆ alkynyl, C₆₋₁₀ aryl, 5 to 10 membered saturated, unsaturated or aromatic heterocycle containing 1 to 4 heteroatoms selected from O, N and S, wherein each of these groups R¹ and R² may itself optionally carry one to three substituents independently selected from halogen, C₁₋₆ alkyl, C₁-₆ alkoxy, -NH₂ and -NO₂ and R³ represents a divalent group selected from C₁₋₆ alkylene, C₂₋₆ alkenylene, and C₂₋₆ alkynylene;
each PDM group represents a photodimerizable group; and
n represents an integer of 2 to 10;
such that the molecular weight of the debonding component of formula (I) is in the range of from 200 g/mol to 3000 g/mol, preferably in the range of from 300 to 1500 g/mol.

3. Adhesive composition according to claim 1, wherein the debonding component is an oligomeric compound which contains two or more repeating units of general formula (II):
-(REP (-LIN-PDM))- (II)
wherein REP represents a repeating unit core moiety derived from a monomeric compound selected from amino acids, saccharides, and vinylacohol;
each LIN group represents a linker moiety independently selected from a covalent bond,-R³-, -O-C(=O)-, -C(=O)-, -O-, -C(=O)-O-, -N(R²)-C(=O)-, -N(R²)- , -C(=O)-N(R²)-, -O-C(=O)-O-, -O-C(=O)-N(R²)-, -N(R²)-C(=O)-O-, -O-C(=N(R²))-, -C(=N(R²))-O-, -O-C(=N(R²))-N(R²)-, -N(R²)-C(=N(R²))-O-,-N(R²)-C(=N(R²))-N(R²)-, and combinations of two or more of these groups, wherein each of the above groups R¹ and R² may be independently selected from hydrogen, C₁₋₆ alkyl, C₂₋₆ alkenyl, C₂₋₆ alkynyl, C₆₋₁₀ aryl, 5 to 10 membered saturated, unsaturated or aromatic heterocycle containing 1 to 4 heteroatoms selected from O, N and S, wherein each of these groups R¹ and R² may itself optionally carry one to three substituents independently selected from halogen, C₁₋₆ alkyl, C₁₋₆ alkoxy, -NH₂ and -NO₂ and R³ represents a divalent group selected from C₁₋₆ alkylene, C₂₋₆ alkenylene, and C₂₋₆ alkynylene;
each PDM group represents a photodimerizable group; and
wherein the molecular weight of the debonding component of general formula (II) is in the range of from 500 g/mol to 6000 g/mol, preferably in the range of from 600 to 4000

4. Adhesive composition according to claim 1, 2 or 3, wherein the PDM group is a group derived from a molecule selected from coumarin, stilbene, anthracene, cinnamic acid, thymine and substituted forms thereof by abstracting one of its hydrogen atoms to provide for the free valency for covalent bonding to the LIN group.

5. Adhesive composition according to any one of claims 1 to 4, wherein the PDM group carries one or more electron withdrawing substituents.

6. Adhesive composition according to any one of claims 1 to 5, wherein the PDM group carries one or more solubilizing groups SOL, wherein SOL is preferably selected from linear or branched alkyl groups with 1 to 24 carbon atoms and poly(C₂₋₆ alkylene ether) groups with 1 to 6 repeating units.

7. Adhesive composition according to claim 2, wherein COR is derived from a tri-, tetra- or hexafunctional alcohol or amine compound and wherein LIN is optionally absent.

8. Adhesive composition according to any one of the preceding claims, wherein PDM is derived from cinnamic acid.

9. Adhesive composition according to claim 8, wherein COR is derived from a tri-, tetra- or hexafunctional alcohol or amine compound, LIN is absent and the PDM group derived from cinnamic acid is directly bonded to COR via ester or amide formation with the hydroxyl or amino groups of COR.

10. Adhesive composition according to claim 3, wherein the debonding component is an oligomer of a repeating unit of general formula (II), wherein REP is derived from lysine and wherein adjacent REP units are linked via peptide bonds, wherein preferably PDM is a the photodimerizable group derived from cinnamic acid and LIN is a covalent amide bond formed between the photodimerizable group derived from cinnamic acid, and the amino group in the side-chain of the repeating unit derived from lysine.

11. Adhesive composition according to any one of the preceding claims 1 to 10, wherein
(a) the adhesive system is a pressure-sensitive adhesive system selected from acrylics, rubbers, silicones, polyurethanes, polyesters, polyethers, EVA systems, sealants, hot-melts, glue and hydrocolloid systems and/or
(b) wherein the adhesive system is based on an adhesive polymer that contains repeating units carrying a PDM group as defined above.

12. Layered product selected from medical patch, medical tape, masking tape and protective tape, which contains a backing layer and an adhesive layer, wherein the adhesive layer comprises the adhesive composition according to any one of claims 1 to 10 and claim 11, embodiment (a) or (b).

13. Method of manufacturing the adhesive composition according to any one of claims 1 to 10 and claim 11, embodiment (a) or (b), which comprises either the step of admixing the debonding component to a pre-existing adhesive system, or the step of mixing the debonding component with one or more of the components of the adhesive system.

14. Method of manufacturing a layered product according to claim 12, which comprises the step of applying a layer of the adhesive composition of the present invention to a backing material, which preferably contains an initial step of coating the adhesive composition of the present invention onto a release liner surface, followed by a step of laminating the surface of the adhesive composition layer opposite to the release liner onto a backing material.

15. Method of debonding the adhesive composition of any one of claims 1 to 10 and claim 11, embodiment (a) or (b) or the layered product of claim 12 from a substrate surface onto which the adhesive composition of any one of claims 1 to 10 and claim 11, embodiment (a) or (b) or the layered product of claim 12 is adhered, which comprises the step of irradiating the adhesive composition of any one of claims 1 to 10 and claim 11, embodiment (a) or (b) or the layered product of claim 12 by irradiation having a wavelength within the range of from 200 nm to 500 nm.
